**Europäisches Patentamt**

**European Patent Office**

**Office européen des brevets**

(19)

(11) Publication number : **0 334 551 B1**

# EUROPEAN PATENT SPECIFICATION

(12)

(45) Date of publication of patent specification :
**09.09.92 Bulletin 92/37**

(21) Application number : **89302599.9**

(22) Date of filing : **16.03.89**

(51) Int. Cl.$^5$ : **A61K 31/28, A61K 9/08, A61K 33/24, A61K 47/00**

(54) **Anti-neoplastic formulations.**

(30) Priority : **20.03.88 IL 85790**

(43) Date of publication of application :
**27.09.89 Bulletin 89/39**

(45) Publication of the grant of the patent :
**09.09.92 Bulletin 92/37**

(84) Designated Contracting States :
**AT BE CH DE ES FR GB GR IT LI LU NL SE**

(56) References cited :
**GB-A- 2 021 946**
**US-A- 4 571 335**
**AMERICAN JOURNAL OF HOSPITAL PHAR-MACY, vol. 44, 1987, YUEN-WAH CHEUNG et al. "Stability of cisplatin, iproplatin,carbopla-tin, and tetraplatin in commonly used intraven-ous solutions" pages 124-130**

(56) References cited :
**DEV. ONCOL., vol. 17, 1984, Martinus Nijhoff Publishing, Boston, The Hague, Dordrecht, Lancaster C.L. LITTERST "PlasmaPharmacokinetics, Urinary Excretion, and Tissue Distribution of Platinum Following IV Administration ofCyclobutanedicar-boxylatoplatinum-II and cis-Platinum to Rab-bits" pages 71-81**

(73) Proprietor : **ABIC LIMITED**
**Industrial Zone Kiryat Nordau POB 8077**
**Natanya (IL)**

(72) Inventor : **Levius, Harold Phillip**
**Ben-Yehuda St. 25/33**
**Natanya (IL)**
Inventor : **Geffen, Yisrael**
**Hagalil St. 42**
**Ra'anana (IL)**
Inventor : **Sklarz, Benjamin**
**Frankfurter St.**
**Petah Tikva (IL)**

(74) Representative : **Sanderson, Laurence Andrew et al**
**SANDERSON & CO. European Patent Attorneys 34, East Stockwell Street Colchester Essex CO1 1ST (GB)**

EP 0 334 551 B1

## Description

This invention relates to anti-neoplastic formulations, and concerns in particular long-term stable aqueous solutions of carboplatin suitable for use by injection.

Carboplatin - the approved name for the platinum compound cis-Diamine[1,1-cyclobutanedicarboxylato-(2-)-O,O']platinum [SP-4-27] - is a well known compound for the treatment (especially by injection of an aqueous solution thereof) of various neoplasms, including advanced ovarian carcinoma, either alone or in combination with other chemotherapeutic agents. At present the compound is provided in the form of a lyophilised powder which has to be reconstituted into an injectable form before use, and this reconstitution may be effected by adding sterile water, or 5% aqueous dextrose, or 0.9% aqueous NaCl, to give the desired injectable solution.

These reconstituted solutions are prepared extemporaneously - that is, just before they are needed - and it is explicitly stated in the prescribing information of one of the main Manufacturers of the lyophilised powder (Bristol-Myers) that the solutions obtained are stable for only 8 hours at room temperature and for only 24 hours under refrigeration (it is recommended that the solution be discarded after these time periods). Unfortunately, the very act of reconstitution can cause problems if improperly performed, and is best avoided. For this reason a (hitherto unobtainable) ready-made stable solution is considered a superior galenic form. Moreover, another important feature is the safety factor - specifically, the protection of personnel during handling of carboplatin. Anti-neoplastic agents often have rather undesirable effects on normal tissue, and it is known from general pharmaceutical practice that handling of ready-made solutions of such agents involves less of a risk of exposure, and so is safer, than does reconstitution of a lyophilised form just prior to use. Consequently, the need for a ready-made, stable, injectable solution of carboplatin, which would overcome the above drawbacks, merits high consideration.

Rather surprisingly it has now been found that these objectives can be attained - and an aqueous solution of carboplatin, stable for at least 18 months at room temperature, can be achieved - by the simple expedient of dissolving unlyophilised carboplatin - that is to say, carboplatin in a form that is not a lyophilisate - in pure, sterile water to form a solution containing at least 10 mg/ml carboplatin.

In one aspect, therefore, the invention provides a stable, aqueous, ready-made injectable solution of carboplatin, characterised in that it is made from unlyophilised carboplatin in pure, sterile water and contains at least 10 mg/ml, preferably 10 to 15 mg/ml carboplatin.

The aqueous solution of the invention may be simply a solution of (powdered) carboplatin in pure, sterile water. However, the solution may include, if desired, additional ingredients, such as a polyol (e.g., mannitol at around 15 mg/ml), or a preservative (e.g., benzyl alcohol). Most desirably, however, the solution will be salt-free, and should specifically be free of sodium chloride and other ionizable salts, for these appear to have a deleterious effect on the long term stability of the solution.

The pH of the carboplatin solution of the invention is preferably above 4, and most preferably from 5 to 7, and usually nothing special has to be done to attain these values. However, if desired the pH can be adjusted, and adjusted above these values.

The carboplatin solutions of the invention can be prepared in any convenient way, but as intimated above a practical method is simply to dissolve the carboplatin in sterile water (for injection) at the desired concentration (e.g., 10 to 15 mg/ml). Naturally, the invention extends to carboplatin solution when thus prepared.

The carboplatin solutions according to the invention may be presented in any appropriate form thus, for example, suitably packed in a sealed container such as a vial or ampoule.

The ready-made carboplatin solutions of the invention can be used safely by nursing staff in the treatment of malignancies.

It will be of interest to compare the simplicity allowed by the invention with the procedures recommended by Bristol-Myers, who sell lyophilised carboplatin under the trade name PARAPLATIN. According to the most recent UK ABPI Data Sheet Compendium 1988-89 (page 281), the reconstitution procedure of the lyophilised product is as follows:

"Reconstitution: Immediately before use, the content of each vial must be reconstituted with Sterile Water for Injection PhEur, 5% dextrose for injection or 0.9% sodium chloride for injection to a final concentration of 10 mg of PARAPLATIN per ml, using a diluent volume of 15 ml.

"Solutions in 5% Dextrose for Injection BP, or Sodium Chloride for injection BP, may be further diluted with the same vehicles originally used for reconstitution, to concentrations as low as 0.5 mg/ml (500 microgram/ml), while solutions originally reconstituted with Sterile water for Injection PhEur may be further diluted with either of these two fluids."

Moreover, having regard to the recommended use of sodium chloride solutions, the contrary conclusions of Cheung Y W et al, of the Analytical and Product Development Section, National Cancer Institute, Bethesda, will also be of interest. They published an article in the Am. J. Hosp. Pharm. 1987, Jan;44(1): p.124-30, on the stability of cisplatin, iproplatin, carboplatin, and tetraplatin in commonly used intravenous solutions, and they came to the conclusion:

"Carboplatin and iproplatin are stable for 24 hours in all the infusion fluids studied, but carboplatin should not be diluted with solutions containing chloride ions because of possible conversion to cisplatin."

The present invention will now be described, though by way of illustration only, with reference to the following Examples.

Example 1

Preparation of 15 mg/ml aqueous carboplatin solutions with and without mannitol

5 g of carboplatin were dissolved in sterile water up to a value of 333 ml (15 mg/ml). From the solution obtained 150 ml were separated (solution B) and 2.25 g of mannitol were added thereto. The other part was kept unchanged (solution A).

The solutions obtained were filled into amber moulded 20 ml vials and sealed with Teflon-lined caps, West 541.

The solutions obtained are shown in Table I below.

TABLE I

| Ingredients | A | B |
|---|---|---|
| carboplatin | 2.75 g | 2.25 g |
| mannitol | - | 2.25 g |
| water for injections to | 183 ml | 150 ml |
| pH | 5.45 | 5.50 |

The above two solutions were stored at room temperature for 15 months, then tested for stability. The results are shown in Table II below.

## TABLE II

| Time and temperature | 15 months at room temperature | |
|---|---|---|
| sub-batch | A | B |
| colour | yellow | yellow |
| clarity | clear | clear |
| pH | 5.64 | 5.35 |
| assay (mg/ml) | 14.8 | 14.9 |
| decomposition | 1.3% | 2.0% |

Notes

1) Assays were by HPLC.

2) Decomposition was estimated as AUC relative to the main peak.

Example 2

Preparation of a 10 mg/ml aqueous carboplatin solution with mannitol

A 10 mg/ml carboplatin solution was prepared from the following ingredients:

| Ingredients | C | |
|---|---|---|
| carboplatin | 3.75 | g |
| mannitol | 3.75 | g |
| sodium chloride | – | |
| water for injections to | 375 | ml |

Method of preparation

The carboplatin was dissolved in some of the water for injections, followed by the mannitol, the formed solution (C) was then made up to volume with water for injections. The pH was not adjusted.

Filling

The solution was filled into 20 ml amber moulded vials and sealed with grey chlorobutyl rubber caps. Stability results on solution C (with mannitol) are shown in Table III below.

TABLE III

| Months | Temp | Colour | Clarity | pH | Assay | Decomposition |
|--------|------|--------|---------|------|-------|---------------|
| 0 | – | none | clear | 5.48 | 10.5 | not detectable |
| 3 | 37°C | none | opalescent | 5.64 | 9.4 | present |
| 6 | RT | none | clear | 5.5 | 10 | not examined |
| 18 | RT | none | clear | 5.48 | 10.1 | – |

Notes

1) Assays were by HPLC in mg/ml.
2) Decomposition was estimated as AUC relative to the main peak.
3) RT is room temperature.

Example 3

Preparation of a 10 mg/ml aqueous carboplatin solution with either mannitol or sodium chloride, and at different pH values

Various solutions were made up from the ingredients (in mg/ml) shown in Table IV below.

TABLE IV

| Ingredients | I | II | III | I | II | III |
|-------------|------|------|------|------|------|------|
| carboplatin | 10 | 10 | 10 | 10 | 10 | 10 |
| mannitol | 10 | 10 | 10 | – | – | – |
| sodium chloride | – | – | – | 9 | 9 | 9 |
| water to (ml) | 1 | 1 | 1 | 1 | 1 | 1 |
| pH | 2.68 | 6.46 | 9.38 | 2.66 | 6.06 | 9.43 |

Method of preparation

1.9 g of carboplatin was dissolved in 180 ml of water for injections. The solution was divided into two equal portions. 0.95 g of mannitol was dissolved in one portion, and 0.855 g of sodium chloride was dissolved in the other portion. Each portion was then made up to a volume of 95 ml with water for injections.

Each of the two solutions was then divided into three equal parts. One of these was made acidic with N HCl, another was made alkaline with N NaOH, and the remaining portion was left unadjusted.

Filling

Each solution was filled into 6 ml clear drawn vials and sealed with Teflon lined caps. The stability test results are shown in Table V below.

5

TABLE V

| Solution type | Mannitol | | | Sodium Chloride | | |
|---|---|---|---|---|---|---|
| Initial pH | 2.68 | 6.46 | 9.38 | 2.66 | 6.06 | 9.43 |

| Time | Temp. | Stability Assay Results | | | | | |
|---|---|---|---|---|---|---|---|
| zero | – | 9.9 | 10.4 | 10.4 | 9.7 | 10.1 | 9.9 |
| 1 day | 60°C | 9.5p | 10.48 | 10.36 | ** | ** | ** |
| 10 days | RT | – | – | – | 8.75p | 8.75p | 9.8p |
| 10 days | 60°C | ** | 10.33 | 10.37 | ** | 7.9p | 7.34p |
| 2 years | RT | 8.71 | 9.67 | 9.65 | ** | ** | ** |

Notes

1) After 2 years the acidic mannitol solution was yellow with a black precipitate. The other two mannitol solutions were clear and colourless.
2) HPLC assay results in mg of carboplatin per ml.
3) ** Decomposition peaks observed in the HPLC assay.
4) p Precipitate.

## Claims

### Claims for the following Contracting States : AT, BE, CH, DE, FR, GB, IT, LU, NL and SE

1. A stable, aqueous, ready-made injectable solution of carboplatin, characterised in that it is made from unlyophilised carboplatin in pure, sterile water and contains at least 10 mg/ml carboplatin.
2. A solution according to Claim 1, containing 10 to 15 mg/ml unlyophilised carboplatin.
3. A solution according to Claim 1 or Claim 2, which includes a polyol and/or a preservative.
4. A solution according to claim 3, wherein the polyol is mannitol, and the preservative is benzyl alcohol.
5. A solution according to any of the preceding Claims, wherein the pH is between 5 and 7.
6. A solution according to any of the preceding Claims, packed in a sealed container.
7. A method of making an aqueous carboplatin solution according to any of the preceding Claims, in which unlyophilised carboplatin is dissolved in pure sterile water free of sodium chloride and any other ionizable salts.

### Claims for the following Contracting States : ES, GR

1. A method of making a stable, aqueous, carboplatin solution ready-made for injection, in which unlyophilised carboplatin is dissolved to a concentration of at least 10 mg/ml in pure sterile water free of sodium chloride and any other ionizable salts.
2. A method according to Claim 1, in which the unlyophilised carboplatin is dissolved to a concentration of from l0 to l5 mg/ml.

3. A method according to Claim l or Claim 2, in which the solution also includes a polyol and/or a preservative.

4. A method according to Claim 3, wherein the polyol is mannitol, and the preservative is benzyl alcohol.

5. A method according to any of the preceding Claims, wherein the pH lies or is adjusted to between 5 and 7.

6. A method according to any of the preceding Claims, wherein the solution thus made is then packed in a sealed container.

**Patentansprüche**

**Patentansprüche für folgende Vertragsstaaten : AT, BE, CH, DE, FR, GB, IT, LU, NL und SE**

1. Stabile, wäßrige, gebrauchsfertige, injizierbare Lösung von Carboplatin, <u>dadurch gekennzeichnet</u>, daß sie aus nicht-lyophilisiertem Carboplatin in reinem, sterilen Wasser hergestellt ist und mindestens 10 mg/ml Carboplatin enthält.

2. Lösung gemäß Anspruch 1, die 10 bis 15 mg/ml nicht-lyophilisiertes Carboplatin enthält.

3. Lösung gemäß Anspruch 1 oder Anspruch 2, welche ein Polyol und/oder ein Ronservierungsmittel einschließt.

4. Löung gemäß Anspruch 3, in welcher das Polyol Mannitol und das Ronservierungsmittel Benzylalkohol ist.

5. Lösung gemäß einem der vorhergehenden Ansprüche, in welcher der pH-Wert zwischen 5 und 7 ist.

6. Lösung gemäß einem der vorhergehenden Ansprüche, die in einem versiegelten Behälter verpackt ist.

7. Verfahren zur Herstellung einer wäßrigen Carboplatinlösung gemäß einem der vorhergehenden Ansprüche, in welchem nichtlyophilisiertes Carboplatin in reinem sterilem Wasser gelöst wird, das frei von Natriumchlorid und anderen ionisierbaren Salzen ist.

**Patentansprüche für folgende Vertragsstaaten : ES, GR**

1. Verfahren zur Herstellung einer stabilen wäßrigen Carboplatinlösung gebrauchsfertig zur Injektion, in welchem nichtlyophilisiertes Carboplatin bis zu einer Konzentration von mindestens 10 mg/ml in reinem sterilem Wasser gelöst wird, das frei von Natriumchlorid und anderen ionisierbaren Salzen ist.

2. Verfahren gemäß Anspruch 1, in welchem das nicht-lyophilisierte Carboplatin bis zu einer Konzentration von 10 bis 15 mg/ml gelöst ist.

3. Verfahren gemäß Anspruch 1 oder 2, in welchem die Lösung auch ein Polyol und/oder ein Ronservierungsmittel einschließt.

4. Verfahren gemäß Anspruch 3, in welchem das Polyol Mannitol und das Konservierungsmittel Benzylalkohol ist.

5. Verfahren gemäß einem der vorhergehenden Ansprüche, in welchem der pH-Wert zwischen 5 und 7 liegt oder eingestellt ist.

6. Verfahren gemäß einem der vorhergehenden Ansprüche, in welchem die so hergestellte Lösung anschließend in einen versiegelten Behälter verpackt wird.

**Revendications**

**Revendications pour les Etats contractant suivants : AT, BE, CH, DE, FR, GB, IT, LI, LU, NL, SE**

1. Solution injectable, stable, aqueuse et prête à l'emploi de carboplatine, caractérisée en ce qu'elle est faite de carboplatine non lyophilisée dans de l'eau pure et stérile, et contient au moins 10 mg/ml de carboplatine.

2. Solution selon la revendication 1, contenant 10 à 15 mg/ml de carboplatine non lyophilisée.

3. Solution selon la revendication 1 ou la revendication 2, qui contient en outre un polyol et/ou un conservateur.

4. Solution selon la revendication 3, dans laquelle le polyol est le mannitol et le conservateur est l'alcool benzylique.

5. Solution selon l'une quelconque des revendications précédentes, dans laquelle le pH se situe entre 5 et 7.

6. Solution selon l'une quelconque des revendications précédentes, logée dans un réservoir scellé.

7. Procédé pour la préparation d'une solution aqueuse de carboplatine selon l'une quelconque des revendications précédentes, dans lequel la carboplatine non lyophilisée est dissoute dans une eau pure, stérile, dépourvue de chlorure de sodium et de tous autres sels ionisables.

**Revendications pour les Etats contractant suivants : ES, GR**

1. Procédé pour la préparation d'une solution aqueuse, stable et prête à l'emploi de carboplatine, dans lequel de la carboplatine non lyophilisée est dissoute dans une eau pure, stérile, dépourvue de chlorure de sodium et de tous autres sels ionisables.

2. Procédé selon la revendication 1, dans lequel la carboplatine non lyophilisée est dissoute jusqu'à une concentration de 10 à 15 mg/ml.

3. Procédé selon la revendication 1 ou la revendication 2, dans lequel la solution contient en outre un polyol et/ou un conservateur.

4. Procédé selon la revendication 3, dans lequel le polyol est le mannitol et le conservateur est l'alcool benzylique.

5. Procédé selon l'une quelconque des revendications précédentes, dans lequel le pH est situé ou ajusté entre 5 et 7.

6. Procédé selon l'une quelconque des revendications précédentes, dans lequel la solution ainsi obtenue est logée dans un réservoir scellé.